Europäisches Patentamt

European Patent Office

Office européen des brevets

(11) Publication number : **0 605 173 A2**

# EUROPEAN PATENT APPLICATION

(21) Application number : **93310371.5**

(22) Date of filing : **21.12.93**

(51) Int. Cl.$^5$ : **C12M 1/40, C12M 1/12**

(30) Priority : **23.12.92 GB 9226820**

(43) Date of publication of application :
**06.07.94 Bulletin 94/27**

(84) Designated Contracting States :
**AT BE CH DE DK ES FR GB GR IE IT LI NL PT SE**

(71) Applicant : **UNIVERSITY OF STRATHCLYDE**
**McCance Building,**
**16 Richmond Street**
**Glasgow G1 1XQ (GB)**

(72) Inventor : **Madhav Vaidya, Atul**
**6/11 Crown Road South**
**Glasgow G12 9DG (GB)**

(74) Representative : **Horner, Martin Grenville et al**
**Cruikshank & Fairweather**
**19 Royal Exchange Square**
**Glasgow G1 3AE Scotland (GB)**

(54) **Hollow fibre reactor.**

(57)    A reactor for conducting an enzyme-catalysed biochemical reaction comprising two immiscible phases, comprises a chamber (2) provided with an inlet (4) and outlet (6) for flowing a first phase through the chamber. A bundle of hollow semipermeable fibres (12) passes through the chamber and a second immiscible phase is flowed through the hollow fibres. A bed of a solid particulate porous material (20) is packed around and between the hollow fibres such as to be in contact therewith. A catalytic enzyme is immobilised on the solid porous material. The first phase permeates through the hollow fibre walls and wets the solid porous material having the enzyme immobilised thereon, where the enzyme-catalysed biochemical reaction occurs. Other reaction materials, such as oxygen, can be supplied via a further bundle of hollow fibres. The reactor is suitable inter alia for hydrolysis, esterification and oxidation reactions.

FIG.1

Jouve, 18, rue Saint-Denis, 75001 PARIS

The present invention relates to a hollow fibre reactor for conducting reactions employing two immiscible phases, usually, though not exclusively two immiscible liquid phases such as an aqueous phase and an organic phase. Usually, the reaction is a biochemical enzyme-catalysed reaction.

The advantages offered by aqueous-organic two phase systems for conducting biocatalytic processes have been outlined in recent reviews (P.J. Halling, Biocatalysis in Multi-phase Reaction Mixtures containing Organic Liquids, Biotech Adv., 5(1) (1987) 47-84; J.S. Dordick, Enzymatic Catalysis in Monophasic Organic Solvents, Enzyme. Microb. Technol., 11 (1989) 194-211; K.D. Mukherjee, Lipase-catalyzed reactions for modification of Fats and other Lipids, Biocatalysis., 3 (1990), 277-293); In many instances, it is necessary to employ two immiscible phases, either because one of the reactants is only appreciably soluble in the aqueous phase, or because some of the reaction products partition poorly into the organic phase.

Emulsification is an obvious technique for increasing the interfacial contact area between the immiscible phases, but there are significant problems in separating the phases after the reaction. High interfacial areas are desirable in order to obtain high reaction and/or mass transfer rates Moreover, it may be necessary to employ emulsifying agents in order to produce the emulsion and the presence of these complicates the post-reaction separation processes. In the case of enzyme-catalysed reactions, the enzymes themselves have some surface activity and thus tend to congregate at the phase interface. This makes recovery of the enzymes difficult and inefficient and leads to wastage of valuable enzyme material.

The use of a membrane which is selectively wetted by one of the two phases has been suggested as an alternative to emulsification. Although this is an attractive method it suffers from a number of potential drawbacks. Firstly, the interfacial area that can be created between the two phases is limited by the geometrical area of the membrane. Secondly, many reactants and products are themselves surface active. Their presence may therefore lead to a change in the wetting characteristics of the membrane material during the course of the reaction. This makes the maintenance of an interface between the phases in the plane of the membrane extremely difficult.

Thus, it has been reported (Ho,M.M. et al J. Am. Oil. Chem. Soc,62:1016) that it is possible to carry out reactions without emulsification by using a microporous polypropylene membrane which is preferentially wetted by the organic phase. Devices of this type have been operated with microporous polytetrafluoroethylene (PTFE) and polyvinylchloride (PVC) membranes, and with symmetric regenerated cellulose ultrafiltration membranes and also with asymmetric ultrafiltration membranes. However, the stable long-term operation of such devices is likely to require pressure control techniques which would be difficult on any industrially significant scale.

It is an object of the present invention to provide a biochemical reactor for conducting reactions employing two immiscible phases which mitigates these problems.

Thus, the present invention provides a reactor for conducting a reaction employing two immiscible phases, which comprises:

- a reaction chamber having an inlet for introducing a first phase into the chamber;
- a series of hollow fibres extending through the chamber, and the series of hollow fibres having an inlet and an outlet thereto for flowing the second phase through the hollow fibres; and
- a solid porous material located in the chamber in contact with outsides of the hollow fibres, the bed of material being adapted to allow a flow of the first phase through the porous material, and the solid porous material being adapted to have immobilised thereon a catalytic substance for reaction involving said first and second immiscible phases.

The use of the solid porous material in contact with the hollow fibres has been found to provide a number of benefits as set out herein.

The reactor may comprise a hollow cylindrical vessel having a bundle of hollow fibres passing therethrough; the flow of first and second phases being essentially concurrent or counter-current. Alternatively, the flow of first and second phases may be substantially at right angles to each other, for example the hollow fibres may be arranged in layers across the chamber and across the direction of flow of the first phase.

The inlet to the chamber (and any outlet therefrom) may be provided with suitable filter means to prevent the solid porous material being washed out of the chamber.

Although the invention is primarily applicable to the reaction of two immiscible phases, for example liquid-liquid phases, or liquid-gas phases, it is also applicable to reactions involving more than two immiscible phases. The reactor is particularly useful for conducting reactions between immiscible organic and aqueous liquid phases. Alternatively, the immiscible phases may act as carriers for other reactive species (e.g. solutes dissolved therein). Either the organic or the aqueous phase may be passed through the hollow fibres, whose characteristics will be arranged to allow passage of the second phase through walls of the hollow fibre to enable the second phase to wet the solid porous material located within the chamber.

The hollow fibres are tubes formed of a material which allows the second phase to pass through the fibre wall. In use, the first phase does not substantially pass through the fibre wall; the first and second immiscible phases remaining separate. Typically, where the hollow fibres are to contain the aqueous phase, the fibres are formed of a hydrophilic water-permeable material such as regenerated cellulose or polyamide. Where the organic phase is inside the hollow fibres, the fibre will normally be formed of a hydrophobic material, such as polypropylene or silicone. The object of the permeable hollow fibres is to provide flow of a second phase through the fibre wall in order to wet the solid porous material. They also allow for diffusion of product in the reverse direction. Thus, the hollow fibres are generally permeable (or so-called semipermeable) membranes having suitable characteristics, such as ultrafiltration membranes, typically having a molecular weight cut off in the region 1,000 to 100,000 Daltons.

The solid porous material is intended to provide two functions. Firstly, the solid porous material should generally be capable of being wetted by the second phase passing through the hollow fibres so as to provide a reservoir of said second phase within its pores and thus a high surface area for contact with the first phase within the chamber. The porous nature of the solid material provides a high surface area to facilitate the reaction. Secondly, the solid porous material provides a substrate for immobilisation (e.g. by adsorption thereon) of a catalytic substance (such as an enzyme) for conducting the reaction. Generally, the solid porous material is in the form of a bed of particles packed into the reactor chamber. The particulate nature of the solid material allows a bulk flow of the first phase through interstices in the bed. However, the packing of particles around the hollow fibres may prove difficult to achieve and runs the risk of damage to the fibres. Therefore, as an alternative, it is possible to generate the solid porous material in situ, for example by the production of an open cell foam material, particularly a polyurethane.

Generally, the solid porous material has a large surface area though its exact properties will depend on the molecular size and nature of the reactants, products and catalytic substance. For example, the pore size of the solid porous material must be sufficient to allow access of reactants to the enzyme or other catalytic substance immobilised therein. Usually, the pore size will be in the region 0.01 to 10 microns. Particulate solid porous materials typically have a particle size of 10 to 100 microns.

Depending on the nature of the second phase, the solid porous material may be hydrophilic or hydrophobic in character. The solid porous material is generally an adsorbent material. In general, gel chromatography packing materials are suitable. Suitable hydrophilic packing materials include a silica, a cellulose, diatomaceous earth, an alumina and cross-linked dextrans, and also a polyurethane. Suitable hydrophobic packing materials include a polypropylene, a polystyrene and other microporous polymers.

For certain biological applications, killed cells may be used along with the solid porous material.

The reactor is particularly suitable as a bioreactor for conducting biochemical reactions, particularly those involving a catalytic substance such as an enzyme immobilised on the solid porous material. However, the catalytic substance may in principal be any type of catalyst.

The enzyme (or other catalytic substance such as a catalytic antibody) may be immobilised on the solid porous material by conventional techniques. For example, the solid porous material may be treated to introduce amino groups, which are covalently linked with glutaraldehyde to amino groups on the enzyme.

However, it is preferred that the enzyme be physically adsorbed onto the solid porous material, such that its activity is maintained.

The reactor of the present invention is particularly suitable for hydrolysis reactions, such as the hydrolysis of fatty esters and oils in the production of fatty acids and alcohols. Particular reactions include hydrolysis of esters and triglycerides catalysed by lipase, esterification of fatty acids to produce mono and di-glycerides catalysed by lipase transfer of alcohol moieties of phospholipids catalysed by phospholipids D, and oxidation of poly-unsaturated fatty acids catalysed by lipoxygenase.

The reactor design may be varied to meet particular circumstances. Thus, a further series (e.g.bundles) of hollow fibres may be disposed in the chamber carrying a third phase. In this way, three phase or multi-step reactions may be carried out in a single reactor. For example, certain enzyme reactions require the supply of further reaction materials such as oxygen, which may be introduced through a separate series of fibres.

Embodiments of the present invention will now be described by way of example only with reference to the drawings wherein;

Figure 1 is a schematic cross sectional elevation of a concurrent biochemical reactor of the present invention;

Figure 2 is a schematic drawing of an element of a further reaction chamber design; and

Figure 3 is an experimental result showing the production of acid by hydrolysis of ester or triglyceride.

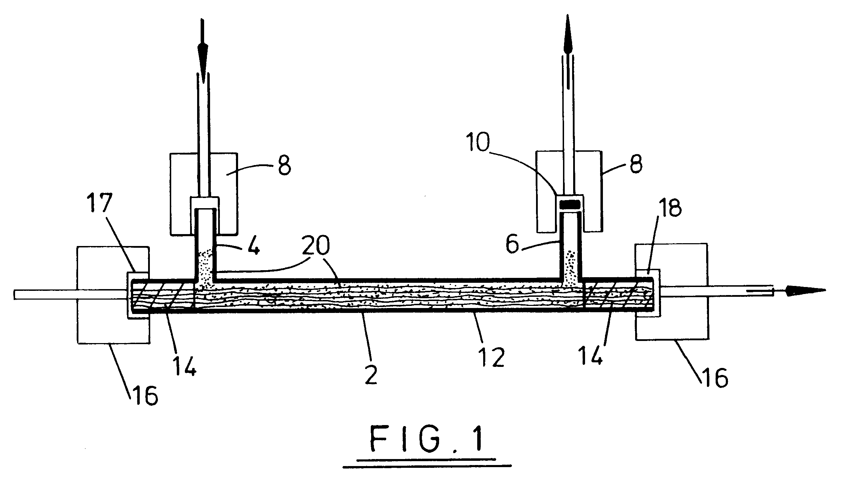

The reaction chamber shown in Figure 1 comprises a cylindrical stainless steel shell 2 provided with inlet 4 and outlet 6 for the first phase. The inlet and outlet are provided with connectors 8 for connection to means for supplying and if necessary recirculating the first phase (not shown). A filter 10 is provided above the outlet 6 to prevent particulate adsorbent material being washed out of the reaction chamber.

A bundle of hollow semipermeable fibres 12 passes through the reaction chamber. The ends of the fibres are potted in conventional fashion in resin 14 into either end of the shell 2 such that the second liquid phase can be passed through the interior lumen of the fibres. Connector pieces 16 are used to act as an inlet manifold 17 and outlet manifold 18 to connect the hollow fibres to an external source of second liquid phase which is circulated through the fibres.

Within the chamber, a bed of a solid particulate microporous adsorbent material 20 is packed around and between the hollow fibres 12 such as to be in contact therewith. The solid adsorbent material acts as a substrate for immobilisation thereon of a catalytic enzyme.

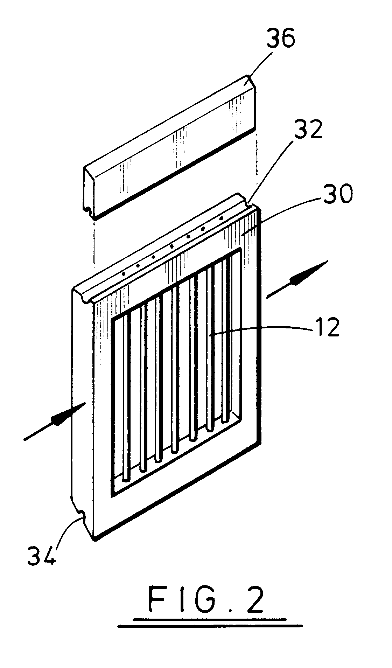

Figure 2 shows an alternative flow chamber arrangement wherein the first phase flows at right angles to the direction of flow of the second phase through the hollow fibres. Thus, frame 30 formed of a plastics material has a series of hollow fibres 12 extending thereacross. Each fibre communicates with a channel 32, 34 at each end thereof. The channel is closed by a further channel piece 36 such as to provide manifolds for feeding second liquid phase through the hollow fibres. The frames may be assembled into layers to give the desired number of hollow fibres. In principal, the flow of the first phase through the chamber can be in either of the two directions at right angles to the fibre direction shown by the arrows.

This arrangement provides simplified packing of the reaction chamber with the solid porous adsorbent material, particularly when this is in particulate form. Thus, the chamber can be built up and filled simultaneously with the addition of solid particulate material and further frames of fibres.

EXAMPLE 1 (Construction and packing of Reactor)

(A) Preparation of Hollow Fiber Reactor

(i) Potting of hollow fibrers

Fibers were packed into a stainless steel shell 2 as shown in Figure 1. Stainless steel shells of 270 mm length were used to pot Cuprophan - regenerated cellulose - fibers obtained from Enka Ltd. Celgard X10 240 - stretched polypropylene - fibers, obtained from Hoechst Celanase Ltd. were potted in shells of 210 cm length. The fibers were introduced into clean, dry shells with a short length (about 25cm) of silicone rubber tubing attached to either end. The number of fibers was determined from their weight. The ends of the silicone rubber tubing were sealed with Hoffman clips and the entire assembly was placed on a turntable rotating at 120 rpm. Araldite CW 1303 resin was mixed with Araldite HY 1303 hardener (both supplied by Ciba Geigy Ltd. Cambridge) and the mixture was injected into the side arms of the shell. Flow of fluid potting compound to the center of the shell was prevented by rotating the turntable for a period of ca 3 hours. The excess potting material, silicone rubber tubing and fibers were cut 8 hours after mixing the potting compound - at which stage the partly cured resin had a 'rubbery' consistency - to minimize damage to the fiber cross section.

(ii) Fluid Connections to Reactors

Nylon end caps were glued to the shells ends with cyanoacrylate adhesive, Superglue 3, (obtained from Bostik Ltd., Leicester). A 2 micron stainless steel sinter, 10 (obtained from Phase Separations Ltd.) was placed in one of the side arm end caps to prevent packing material from being washed out by the shell side fluid. 1/16" stainless steel tubing about 50 mm long was fixed into the end caps with the same cyanoacrylate adhesive.

A total of four reactor shells were prepared - two with each type of membrane. The relevant data for each reactor are given in Table 1.

## Table 1. Number & types of fibers packed in reactor shells.

| Reactor | Fiber Type | Number of fibers | Membrane Area[a] |
|---------|------------|------------------|------------------|
| R1 | Cuprophan | 552 | $875cm^2$ |
| R2 | Cuprophan | 541 | $856cm^2$ |
| R3 | Celgard | 480 | $615cm^2$ |
| R4 | Celgard | 507 | $650cm^2$ |

[a] The area has been calculated on the basis of the measured outside diameters of the fibers -(220 microns for Cuprophan and 240 microns for Celgard).

Details of the fibers are as follows:

Celgard hollow fibers

| | |
|---|---|
| Outside diameter | : 240 microns |
| Wall thickness | : 30 microns |
| Porosity | : 30% |
| Surface pore size | : 0.05 x 0.15 microns |
| Bulk pore size | : 0.075 microns. |

Cuprophan hollow fibers

| | |
|---|---|
| Outside diameter | : 220 microns |
| Wall thickness | : 20 microns |

Pore size etc. - not specified - probably below 2nm.

### (B) Packing Preparation

XAD-8, a non-ionic, modified polystyrene adsorbent, obtained from Sigma Chemical Company, Poole and Accurel polypropylene microstructures supplied by Akzo GmbH, West Germany, were used as packing materials. The physical characteristics of these packings are given in Table 2.

Table 2. Properties of XAD-8 and Accurel packings.

| Adsorbent | Surface Area | Avg.Pore Diameter | Mesh Size |
|-----------|--------------|-------------------|-----------|
| XAD-8 | $160m^2/g$ | 22.5 nm | 20-60 |
| Accurel | $90m^2/g$ | 01.1 micron | >65 |

Details of the packing materials are as follows.

Accurel

| | |
|---|---|
| Surface area | : 90 $m^2/g$ |
| Pore size | : 0.1 - 1 micron |
| Mesh size | : >65 |

Void volume     : 72%

<u>XAD-8</u>

Surface area       : 160 m$^2$/g
Average pore size   : 22.5nm
Mesh size (wet) 20-60.
Both materials were prepared for packing into the reactor shells as follows:
(i) The granular packing was sieved to remove fines and only particles retained by a sieve with a 200 microns opening were used for enzyme immobilisation and packing;
and (ii) 10 g of sieved particles were washed with 100 ml of ethanol under water suction followed by 1 liter of distilled water. The washed packing was allowed to dry under water suction for a period of 20 minutes and then stored in sealed vials.

### (C) Enzyme Immobilization

#### (i) <u>Buffer Solution Preparation:</u>

A 0.1M phosphate buffer was made by adjusting the pH of a 0.1M aqueous solution of sodium dihydrogen phosphate with concentrated aqueous NaOH. The solution was used for dissolving enzyme for immobilisation. The pH of the buffer was adjusted to 8.2 at 25°C.

#### (ii) <u>Enzyme Solution Preparation:</u>

508mg of Lipase Candida Cylindracea, L1754 from Sigma Chemical Co. Ltd. was used. 508mg of enzyme was dissolved in 316.7g of buffer to give a solution containing 1.793mg/g of enzyme. The initial activity of the solution was found to be 23.23U/g - using the method described below.

#### (iii) <u>Estimation of Enzyme Activity</u>

The activity of the enzyme solutions was determined pH statically at pH 8.0, 25°C. One unit of enzyme activity was defined as that amount of enzyme required to release butyric acid by hydrolysis of tributyrin at an initial rate of 1 micromole/min. The assay was done with 4ml of tributyrin emulsion, containing 3% by weight of tributyrin. The emulsion was made up with equal volumes of buffer and a saturated Gum Arabic solution in the same buffer. Gum Arabic, G 9752, and Tributyrn, T 5142, were both obtained from Sigma Chemical Co.

#### (iv) <u>Adsorption of Enzyme</u>

10g of the packing material - treated as described earlier - was taken in a stoppered conical flask and 150g of enzyme solution was added to the flask. The contents of the flask were stirred on a magnetic stirrer at room temperature for a period of six hours. At the end of this period the solution was filtered on a sintered glass funnel under water suction and the packing material retained on the funnel was washed with 500 ml of buffer solution. The filtrate and washings were collected and a sample was taken to determine residual enzyme activity. The packing was dried under water suction for a period of 20 minutes and then stored in sealed vials at -40°C with indicating silica gel as dessicant.
(v) The residual enzyme activity was found to be 0.9842U/g of total washings obtained from the solution used to treat XAD-8. The maximum amount of enzyme immobilized per gram of adsorbent was hence 284.54U. The washings obtained after treating Accurel were found to have no enzyme activity. Consequently, the maximum amount of enzyme immobilized per gram of Accurel was estimated to be 348.5U.

### (D) Packing of Reactor Shells

A polypropylene slurry reservoir was fitted onto the uncapped side arm of the reactor shell - prepared as described in (A) & (B) above. The reactor shell, with the reservoir attached, was clamped in an orbital shaker.
A syringe pump was used to force distilled water through the fiber lumen - at a rate of 200 microlite/min - to prevent collapse of the fibers during the packing operation. A 1% (w/w) slurry of the packing material in hexane with enzyme immobilized on it, as described earlier - was poured into the slurry reservoir which was then pressurized to 1 bar. The entire reactor assembly, along with the slurry reservoir, was agitated by operating the orbital shaker at a speed of 120 rpm.

All the reactor shells were packed in this manner. The type and quantity of packing used with each shell is summarized in Table 3.

Table 3. Type and weight of packing used in reactors.

| Reactor | Packing Type | Weight of Packing |
|---------|--------------|-------------------|
| R1 | Accurel | 7.6g |
| R2 | XAD-8 | 7.2g |
| R3 | Accurel | 4.1g |
| R4 | XAD-8 | 3.9g |

The slurry reservoir was replaced by a nylon end cap, with a 50mm length of 1/16" (1.6mm) SS tubing attached, as described earlier. Distilled water was flushed through the shell and lumen of the reactors for a period of 48 hours to remove all traces of hexane. This operation was carried out at 5°C in order to minimize deactivation of the enzyme.

REACTOR OPERATION

Experiment 1:

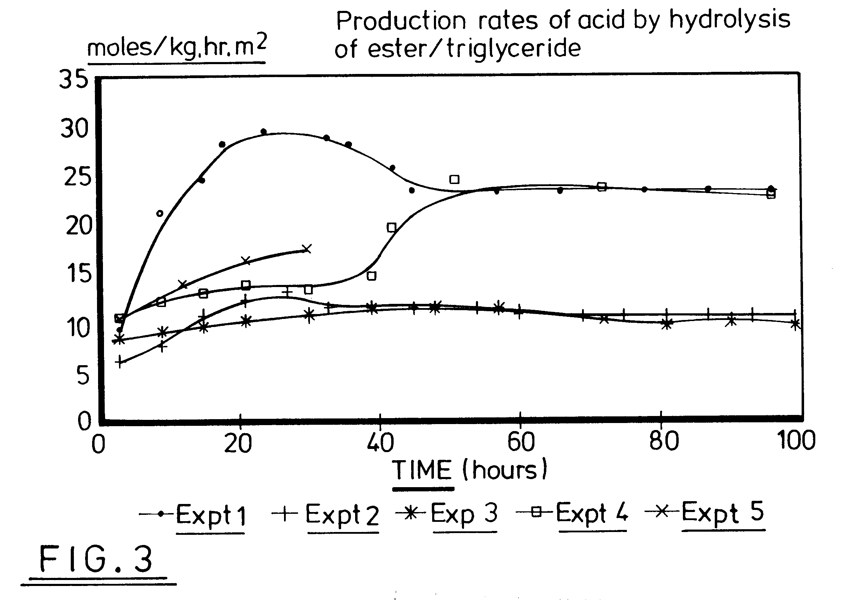

Buffer solution was pumped through the lumen of the hollow fibres of reactor R2. Lauric acid ethyl ester was forced through the shell of the reactor at a rate of 0.94cc/hr. In a separate experiment the void volume of the packing was estimated to be 2.81cc. Thus a residence time of three hours obtained for the shell side fluid. Samples of the effluent organic phase were collected at three hour intervals over a period of one week. The acid content of the samples was measured on a gas chromatograph after derivatization of the acid. The acid production rate over the first 100 hours is shown in Figure 3. The production rate was found to remain nearly constant at ca 2 millimoles/hr/g of packing for the rest of the week.

Experiment 2:

Buffer solution was pumped through the shell of reactor R4. Ethyl laurate was fed to the fiber lumen with a syringe pump at a constant rate of 0.38cc/hr giving a residence time of three hours, as with the reactor in experiment 1. The reactor was run for a period of 100 hours. The production rate of lauric acid over this period was determined and is shown in Figure 3.

Experiment 3:

Buffer solution was pumped through the lumen of reactor R1. Ethyl laurate was pumped through the packing at a rate of 2.36cc/hr. In a separate experiment the void volume of the Accurel packing was estimated to be 0.93cc/g. Consequently, the ester was estimated to have a residence time of 3 hours. The reactor was run for a period of 200 hours. The production rate of lauric acid during the first 100 hours is shown in Figure 3. A slight decline, ca 7%, was observed over the 100-200 hour period.

Experiment 4:

Buffer solution was pumped through the shell of reactor R3. Ethyl laurate was pumped through the fiber lumen at a rate of 0.40cc/hr, giving a residence time of 3 hours for the ester. The reactor was run for a period of 120 hours. The production rate of lauric acid over this period is shown in Figure 3.

Experiment 5:

Reactor R2, used in experiment 1, was cleaned to deactivate enzyme already present on it using the following procedure:

(i) Distilled water was passed through the packing at 40°C over a period of 48 hours at 1cc/hr.

(ii) This was followed by a 1mg/g solution of pepsin, P7012 Sigma, in 0.1M HC1 at room temperature for a 24 hour period, also at 1cc/hr.

(iii) Distilled water was then pumped through the packing at the same flow rate for a further 100 hours at room temperature.

During each of the three steps, described above, a water flow rate of 1cc/hr was maintained through the fiber lumens to avoid collapse of the fibers under the pressure resulting from shell side flow.

The cleaned reactor was placed in a water bath maintained at 5°C and a 3mg/g solution of lipase was pumped through the packing at a rate of 1.5cc/hr. The enzyme solution and the depleted effluent solution were also maintained at 5°C during this operation, in order to minimize deactivation of the enzyme. This 'in situ' immobilization was carried out for a period of 48 hours. At the end of this period buffer solution was pumped through the packing at a rate of 1cc/hr for a further 10 hours. The entire effluent was assayed for enzyme activity, as was the original enzyme solution. From these assays it was estimated that the 172U of enzyme had been adsorbed per gram of packing in the column.

## Experiment 6:

Reactor R2 regenerated as described in experiment 5, was used for the lipolysis of Olive Oil. The high viscosity of olive oil meant that it was necessary to have a lower flow rate of oil in the shell. This lower flow rate, 0.8cc/hr, resulted in a higher residence time of 3.51 hours. Buffer solution was fed to the fiber lumen and the effluent organic phase was collected continuously, with stirring. A 2g sample of oil was withdrawn at intervals of 3.5 hours, diluted in THF and titrated against a 0.1M t-butyl ammonium hydroxide (product number 19,187, Aldrich Chemical Co. Ltd. Gillingham) in toluene/methanol. A solution of methylene blue in methanol was used as the end point indicator. This reactor was run for a period of 28 hours. The rate of production of fatty acids over this period is shown in Figure 3. The results of all these experiments are summarized in Table 4.

Table 4

| Reactor I.D. | Experiment No. | Substrate | Acid Production Rate |
|---|---|---|---|
| R1 | 3 | Ethyl laurate | 11.43 |
| R2 | 1 | Ethyl laurate | 23.36 |
| R3 | 4 | Ethyl laurate | 22.76 |
| R4 | 2 | Ethyl laurate | 10.77 |
| R2 | 6 | Olive oil | 16.35 |

Acid production rates are given in moles/kg.hr.$m^2$ based on the mass of packing used and the available membrane surface area.

## Claims

1. A reactor for conducting a reaction employing two immiscible phases, which comprises:
   - a reaction chamber (2) having an inlet (4) for introducing a first phase into the chamber;
   - a series of hollow fibres (12) extending through the chamber, and the series of hollow fibres having an inlet (17) and an outlet (18) thereto for flowing the second phase through the hollow fibres; and
   - a solid porous material (20) located in the chamber in contact with outsides of the hollow fibres, the bed of material being adapted to allow a flow of the first phase through the porous material, and the solid porous material being adapted to have immobilised thereon a catalytic substance for reaction involving said first and second immiscible phases.

2. A reactor according to claim 1 wherein the reaction chamber is further provided with an outlet (6), so as to allow the first phase to be flowed through the chamber.

3. A reactor according to any preceding claim wherein the hollow fibres are formed of a hydrophilic water-permeable material.

4. A reactor according to any preceding claim wherein the hollow fibres are formed of a hydrophobic material which is permeable to organic liquid.

5. A reactor according to any preceding claim wherein the hollow fibres allow diffusion of a reaction product of said reaction from the chamber into the second phase within the hollow fibres.

6. A reactor according to any preceding claim wherein the hollow fibres are formed of a membrane have a molecular weight cut-off in the region 1000 to 100,000 Daltons.

7. A reactor according to any preceding claim wherein the solid porous material is a bed of a particulate material.

8. A reactor according to any of claims 1 to 6 wherein the bed of solid porous material is an open-cell foam material.

9. A reactor according to any preceding claim wherein the catalytic substance is an enzyme.

10. A reactor according to claim 9 wherein the enzyme is chemically linked to the solid porous material.

11. A reactor according to claim 9 wherein the enzyme is physically adsorbed onto the solid porous material.

12. A reactor according to any preceding claim which further comprises a further series of hollow fibres extending through the chamber for supplying further reaction materials for said reaction.

13. A method of conducting a reaction employing two immescible phases which comprises:
    - introducing a first phase into a chamber through an inlet thereto;
    - flowing a second phase through a series of hollow fibres extending through the chamber;
    - a solid porous material being located in the chamber in contact with outsides of the hollow fibres, and the first phase being allowed to flow through the porous material, and a catalytic substance being immobilised on the solid porous material, such that a reaction involving said first and second immiscible phases is conducted.

F I G . 1

FIG. 2

Production rates of acid by hydrolysis of ester/triglyceride

moles/kg.hr.m$^2$

TIME (hours)

——•—Expt 1  —+—Expt 2  —*—Exp 3  —▫—Expt 4  —✳—Expt 5

FIG. 3

EP 0 605 173 A2